# EUROPEAN PATENT SPECIFICATION

(11) **EP 1 998 747 B1**
(45) Date of publication and mention of the grant of the patent: **31.01.2018**
(21) Application number: 07712936.9
(22) Date of filing: 21.03.2007
(51) Int. Cl.: A61K 8/98, A61Q 19/00, A61Q 19/08

(54) **USE OF FERMENTED MILK PRODUCT FOR SKIN TREATMENT**
VERWENDUNG VON FERMENTIERTEM MILCHPRODUKT ZUR BEHANDLUNG DER HAUT
UTILISATION DE PRODUIT LAITIER FERMENTE POUR TRAITEMENT DE LA PEAU

(30) Priority: 24.03.2006 GB 0605949
(43) Date of publication of application: 10.12.2008
(73) Proprietor: Givaudan S.A., 1214 Vernier (CH)
(72) Inventor: LIEUREY, Severine, Kent TN23 5R7 (GB); WATKINS, Stephen, Kent TN24 9HZ (GB)
(74) Representative: Global Patents
(86) International application number: PCT/GB2007/001003
(87) International publication number: WO 2007/110589

(56) References cited:
- EP-A1- 0 315 541
- EP-A1- 1 541 116
- WO-A-00/27352
- WO-A-01/91588
- WO-A-03/070208
- WO-A-2005/087190
- WO-A-2005/092121
- US-A1- 2006 247 162

## Description

### Technical Field

The present invention relates to the use of a fermented milk product comprising non-hydrolysed whey proteins for treatment of skin, especially human skin.

### Background and Prior Art

Milk and dairy products have been used in cosmetic skin care applications for many hundreds of years as a way to add moisture and fats to the skin in order to condition and moisturise. Fermented milk (e.g. yoghurt) has also been used historically for skincare applications. Such yoghurts are typically high in casein content.

It is well documented that exposure to sunlight damages the skin structure. In response to this damage the skin repairs itself through the rapid production of collagen and other associated dermal components such as polysaccharides. Unfortunately, this rapid formation can result in an unstructured 'mess' of fibres, a condition termed elastosis. It is common for ingredients that show the ability to structure collagen to also trigger collagen synthesis, a result that is not always desirable.

WO 2004/098632 (Snow Brands) discloses cosmetic compositions containing inhibitors which promote skin collagen formation, containing milk basic protein fractions or their hydrolysates.

US 6,203,805 (Color Access) discloses use of whey protein to enhance production of collagen in skin.

EP 0046326 A discloses use of non-hydrolysed whey products in cosmetics.

WO 00/27352 discloses a topical composition comprising collagen enhancing effective amounts of a whey protein, a retinoid, a vitamin E or derivatives thereof and an ascorbic acid or derivatives thereof in specific amounts. The whey protein is denatured by the process of treating milk to make cheese, as well as other pasteurization processes, and does not contain free amino acids, caseins, casein-bound calcium and phosphate, fat and fat soluble vitamins.

### Summary of the Invention

The present invention relates to the use of a fermented milk product according to claim 1 comprising non-hydrolysed whey proteins which is substantially free of casein proteins for the purpose of improving skin firmness when topically applied to skin.
The particular fermented milk products used according to the invention have been found to be effective at structuring collagen without promoting collagen synthesis. This surprising and unusual property has the result that skin firmness improves when the product is topically applied to the skin.
Substantially free of casein proteins means that the product comprises less than 5 wt%, preferably less than 3 wt%, more preferably less than 2 wt%, or even less than 1 wt% of casein proteins.
Thus, the invention relates to the use of a fermented milk product comprising non-hydrolysed whey proteins which is substantially free of casein proteins for the purpose of structuring collagen without promoting collagen synthesis when topically applied to skin.
The novel property of the fermented milk products makes them particularly suitable for use on damaged skin when the skin will naturally generate collagen as part of the repair process. Thus, in a further aspect, the invention relates to the use of a fermented milk product comprising non-hydrolysed whey proteins which is substantially free of casein proteins for application on damaged skin for the purpose of collagen structuring.

Preferably, the milk product contains low or no milk fats, typically achieved by fermenting skimmed milk. Low or no milk fats generally means that the milk product contains no more that 10 wt % milk fats, preferably no more than 5 wt % milk fats, more preferably no more than 2 wt % milk fats, or even containing zero milk fats.

The fermented milk product is a spray dried modified yoghurt product consisting of the selected milk fractions: whey, whey and non-fat dry milk (2%) that are fermented with the classic yoghurt bacteria *Streptococcus thermophilus* and *Lactobacillus bulgaricus.* The invention conveniently uses modified yoghurt, modified by treatment to remove substantially all of the casein proteins. Such modification has the benefits of producing material that is less allergenic (as allergenic effects are generally due to casein proteins), also removing some of the fat content, and producing material that is less prone to smelling (as rancid "off" smells associated with milk and milk-based products are generally due to casein proteins and/or fat). Yoghurt may also be further modified by addition of whey proteins to compensate for removal of casein proteins. Such modifications are suitably performed before the fermentation stage in the conventional process for producing yoghurt.

The fermented milk product is used in dried condition for inclusion in a composition (e.g. in the form of a powder or granules) rather than in liquid condition. Use of a dried fermented milk product compared to one in liquid condition as an ingredient in a topically applied product has certain practical benefits, in particular in terms of a longer shelf life of the dried ingredient, lower content of micro-organisms and avoidance of risk of microbial growth. Material in dry condition can also be incorporated into powdered or anhydrous products.

The fermented milk product is conveniently dried by a spray drying technique.
Suitable drying techniques are well known to those skilled in the art.

According to the present invention, a spray dried modified yoghurt product known by the Trade Mark Yogurtene and available from Quest International as a food supplement is used. Yogurtene consists of selected milk fractions: whey, whey concentrates (components of the serum phase of milk) and non-fat dry milk (2%), that are fermented with classic yoghurt bacteria (*Streptococcus thermophilus* and *Lactobacillus bulgaricus*). Whey is the preferred protein source, because casein proteins are associated with allergenic reactions. Once fermentation is complete, the liquid yoghurt is spray dried to produce a free-flowing, slightly hygroscopic, low odour white to off-white powder.

The fermented milk product is desirably substantially free of live bacteria, i.e. it contains no more than 1000 cfu/g, preferably no more than 500 cfu/g, more preferably no more than 200 cfu/g, or even zero live bacteria. Bacteria can be conveniently destroyed by heat treating the milk product in a manner known to a person skilled in the art.

The fermented milk product used in the present invention is typically applied to the skin, in particular human skin, in a topically applied product. Examples of suitable products are body wash compositions, soap-based products, skin creams and lotions, sunscreens and after-sun products, and in particular, anti-ageing compositions which may also include components for treating cellulite, sagging, wrinkling, age spots and the like. Other suitable products are cosmetics such as lipsticks, foundations and the like.

The amount of fermented milk product in a topically applied product will vary depending upon the application but will generally fall within the range of from 0.005 to 20 wt %.

The invention will now be further described, by way of illustration, in the following examples:

### Example 1: Collagen Fibre Organisation

Collagen is a major constituent of the human skin and accounts for a high proportion of the skin's elasticity and physical properties. When collagen is incubated in vitro with normal human dermal fibroblasts (NHDF), the NHDF will spontaneously bind to the soluble collagen and organise it into a structured lattice. The resulting structure visibly shrinks as this process occurs, and the level of shrinkage is directly proportional to the amount of structuring that occurs.

The rate of collagen organisation can be influenced by the addition of different ingredients into the system. Therefore, it can be shown that some materials will enhance the rate of the lattice formation and so could have a 'firming' effect if applied topically to the skin.

A collagen solution was prepared in vitro (1.3mg/ml) and incubated with NHDF (100,000 NHDF/ml) over a 168-hour period (*control*). The amount of 'lattice shrinkage' was monitored at 24 hours, 72 hours, 144 hours and 168 hours. Over this period, the same concentration of collagen / NHDF was cultured with Yogurtene™ at 0.08mg/ml, 0.4% mg/ml and 2mg/ml. A positive control, Transforming Growth Factor (beta-TGF), was used to compare to the lattice enhancing effects of Yogurtene™. Each test was conducted in triplicate and the mean shrinkage calculated.

Table 1 (below) shows the surface area of the collagen solution as measured over the experiment.

**Table 1**

| | | 24hr incubation | | 72hr incubation | | 144hr incubation | | 168hr incubation | |
|---|---|---|---|---|---|---|---|---|---|
| Treatment | | Surface Area (mm²) | mean | Surface Area (mm²) | mean | Surface Area (mm²) | mean | Surface Area (mm²) | mean |
| Control | - | 590.70 | 557.20 | 464.70 | 443.54 | 332.10 | 328.57 | 264.39 | 266.50 |
| | | 549.52 | | 433.36 | | 309.79 | | 258.96 | |
| | | 531.37 | | 432.56 | | 343.81 | | 276.14 | |
| beta-TGF | 10ng/ml | 589.89 | 586.75 | 456.84 | 436.64 | 192.89 | 193.24 | 107.19 | 110.27 |
| | | 580.88 | | 416.06 | | 194.06 | | 121.69 | |
| | | 589.49 | | 437.03 | | 192.76 | | 101.92 | |
| Yogurtene | 2mg/ml | 562.61 | 560.72 | 419.74 | 411.62 | 301.20 | 297.58 | 223.45 | 213.82 |
| | | 545.66 | | 394.42 | | 292.20 | | 215.04 | |
| | | 573.89 | | 420.69 | | 299.34 | | 202.96 | |
| | 0.4mg/ ml | 559.88 | 549.48 | 450.62 | 442.17 | 336.42 | 332.79 | 243.58 | 243.46 |
| | | 525.61 | | 422.87 | | 319.17 | | 234.32 | |
| | | 562.96 | | 453.02 | | 342.78 | | 252.47 | |
| | 0.08mg/m 1 | 529.16 | 517.51 | 430.42 | 416.94 | 333.33 | 321.89 | 264.25 | 264.69 |
| | | 511.60 | | 406.61 | | 309.85 | | 258.10 | |
| | | 511.77 | | 413.79 | | 322.48 | | 271.73 | |

Thus Yogurtene™ shows noticeable collagen structuring.

### Example 2: Dermal Extracellular Matrix Synthesis

Normal human dermal fibroblasts (NHDF) were cultured in-vitro with radio labelled [³H] proline. Collagen is a proline-rich protein. The level of radio labelled proline found in proteins, following culturing of the NHDF, is a measure of the rate of collagen synthesis. Yogurtene™ can be added to the cell culture to measure the effect of Yogurtene™ on the rate of proline uptake (collagen synthesis).

The study investigated the increase in radio labelled proline found in: (i) soluble/secreted proteins, and (ii) intracellular and extra-cellular matrix (ECM) proteins.

Beta-Transforming Growth Factor (β-TGF) and ascorbic acid are known collagen synthesis promoters and were used as controls in both studies. Tables 2 and 3 show the levels of proline found in the associated proteins.

**Table 2: Proline incorporation into soluble proteins**

| **Treatment** | **Concentration** | **Count/min** |
|---|---|---|
| Control | - | 8180 |
| β-TGF | 10 ng/ml | 17303 |
| Ascorbic acid | 20 micro g/ml | 17918 |
| Yogurtene™ | 2 mg/ml | 8759 |
| | 0.4 mg/ml | 8589 |
| | 0.08 mg/ml | 8363 |
| | 0.016 mg/ml | 7992 |

**Table 3: Proline incorporation into intracellular & ECM layer proteins**

| **Treatment** | **Concentration** | **Count/min** |
|---|---|---|
| Control | - | 10325 |
| β-TGF | 10 ng/ml | 18386 |
| Ascorbic acid | 20 micro g/ml | 12764 |
| Yogurtene™ | 2 mg/ml | 11421 |
| | 0.4 mg/ml | 11917 |
| | 0.08 mg/ml | 10418 |
| | 0.016 mg/ml | 11023 |

This data demonstrates that Yogurtene™ does not increase the synthesis of collagen.

## Claims

1. Non-therapeutic use of a fermented milk product comprising non-hydrolysed whey protein which is substantially free of casein proteins for the purpose of structuring collagen without promoting collagen synthesis when topically applied to skin, wherein the milk product comprises yoghurt or yoghurt-derived material, wherein the milk product is a spray dried modified yoghurt product consisting of the selected milk fractions: whey, whey concentrates and non-fat dry milk (2%) that are fermented with the classic yoghurt bacteria *Streptococcus thermophilus* and *Lactobacillus bulgaricus.*

2. The use of a fermented milk product according to claim 1, wherein the milk product comprises no more than 10 wt% milk fats.

3. The use of a fermented milk product according to any one of the preceding claims, wherein the milk product is in dried condition.

4. The use of a fermented milk product according to any one of the preceding claims, wherein the milk product is substantially free of live bacteria.

5. The use of a fermented milk product according to any one of the preceding claims, wherein the milk product is a spray-dried modified yoghurt product substantially free of live bacteria and containing no more than 10 wt% milk fats.

6. The use of a fermented milk product according to any one of the preceding claims, wherein the fermented milk product is applied in a topically applied product.

## Patentansprüche

1. Nichttherapeutische Verwendung eines fermentierten Milchprodukts, das nichthydrolysiertes Molkeprotein umfasst, das im Wesentlichen frei von Kaseinproteinen ist, zum Zweck der Strukturierung von Collagen ohne Fördern der Collagensynthese, wenn topisch auf Haut aufgebracht, wobei das Milchprodukt Joghurt oder Joghurt-abgeleitetes Material umfasst, wobei das Milchprodukt ein sprühgetrocknetes modifiziertes Joghurtprodukt ist, das aus den ausgewählten Milchfraktionen: Molke, Molkekonzentrate und fettfreie Trockenmilch (2 %), die mit den klassischen Joghurtbakterien *Streptococcus thermophilus* und *Lactobacillus bulgaricus* fermentiert sind, besteht.

2. Verwendung eines fermentierten Milchprodukts gemäß Anspruch 1, wobei das Milchprodukt nicht mehr als 10 Gew.-% Milchfette umfasst.

3. Verwendung eines fermentierten Milchprodukts gemäß einem der vorstehenden Ansprüche, wobei das Milchprodukt in getrocknetem Zustand vorliegt.

4. Verwendung eines fermentierten Milchprodukts gemäß einem der vorstehenden Ansprüche, wobei das Milchprodukt im Wesentlichen frei von lebenden Bakterien ist.

5. Verwendung eines fermentierten Milchprodukts gemäß einem der vorstehenden Ansprüche, wobei das Milchprodukt ein sprühgetrocknetes modifiziertes Joghurtprodukt ist, das im Wesentlichen frei von lebenden Bakterien ist und nicht mehr als 10 Gew.-% Milchfette enthält.

6. Verwendung eines fermentierten Milchprodukts gemäß einem der vorstehenden Ansprüche, wobei das fermentierte Milchprodukt in einem topisch aufgebrachten Produkt aufgebracht wird.

## Revendications

1. Utilisation non thérapeutique d'un produit de lait fermenté comprenant une protéine de lactosérum non hydrolysée qui est sensiblement dépourvue de protéines de type caséine, dans le but de structurer du collagène sans favoriser la synthèse de collagène lors d'une application topique à la peau, où le produit de lait comprend du yaourt ou un matériau dérivé de yaourt, où le produit de lait est un produit de yaourt modifié et séché par pulvérisation constitué des fractions de lait sélectionnées : lactosérum, concentrés de lactosérum et extrait sec dégraissé du lait (2%) qui sont fermentés avec les bactéries de yaourt classiques *Streptococcus thermophilus* et *Lactobacillus bulgaricus.*

2. Utilisation d'un produit de lait fermenté selon la revendication 1, dans laquelle le produit de lait comprend au plus 10% en poids de matières grasses de lait.

3. Utilisation d'un produit de lait fermenté selon l'une quelconque des revendications précédentes, dans laquelle le produit de lait se trouve sous un état séché.

4. Utilisation d'un produit de lait fermenté selon l'une quelconque des revendications précédentes, dans laquelle le produit de lait est sensiblement dépourvu de bactéries vivantes.

5. Utilisation d'un produit de lait fermenté selon l'une quelconque des revendications précédentes, dans laquelle le produit de lait est un produit de yaourt modifié et séché par pulvérisation, sensiblement dépourvu de bactéries vivantes, et contenant au plus 10% en poids de matières grasses de lait.

6. Utilisation d'un produit de lait fermenté selon l'une quelconque des revendications précédentes, dans laquelle le produit de lait fermenté est appliqué dans un produit appliqué par voie topique.
